# EUROPÄISCHE PATENTANMELDUNG

(11) **EP 1 593 385 A1**
(43) Veröffentlichungstag der Anmeldung: **09.11.2005**
(21) Anmeldenummer: 04011015.7
(22) Anmeldetag: 07.05.2004
(51) Int. Cl.: A61K 38/16, C12Q 1/68, A61P 31/12

(54) **Verwendung von Histonen zur Frühdiagnose und/oder Präventivtherapie virusinfizierter lebender Zellen und ein Biochip zur Durchführung der Diagnose**

(71) Anmelder: SYMBIOTEC Gesellschaft zur Forschung und Entwicklung auf dem Gebiet der Biotechnologie mbH, 66123 Saarbrücken (DE); Philadelphia Health and Education Corporation, Philadelphia, PA 19102 (US)
(72) Erfinder: Class, Reiner Dr.Ph.D., 66740 Salouis (DE)
(74) Vertreter: Pätzold, Herbert

(57) **Zusammenfassung**

Die Erfindung eines biologisch aktiven Wirkstoffs, insbesondere zur Frühdiagnose und/oder Präventivtherapie von virusinfizierten lebenden Zellen, wobei seine Wirksamkeit selektiv gegen die Zellmembranen der Virus-infizierten Zellen gerichtet ist, die nach dem Virusbefall in charakteristischer Weise modifiziert sind, wobei der Wirkstoff wenigstens einen Bestandteil beinhaltet, der ausgewählt ist aus einer Gruppe von Stoffen bestehend aus Histonen, kovalent modifizierten Histonen, histonähnlichen Polypeptiden und biologisch aktiven Sequenzen der Histone und histonähnlichen Peptide.

Bei dem Wirkstoff handelt es sich beispielsweise um rekombinantes humanes Histon H1 oder wenigstens ein Histon H1-Subtyp (H1.0, H1.1, H1.2, H1.3, H1.4, H1.5, H1.t, H1.x) oder dessen aktiver Abschnitt. Dabei kann die ausreichende Größe der biologischen Aktivität zur Abtötung einer virusinfizierten Zelle an ihrer modifizierten Zellmembran durch die Kooperativität mit gleichen oder unterschiedlichen aktiven Wirkstoffbestandteilen erzielbar sein, die miteinander einen biologisch wirksamen Komplex mit gesteigerter biologischer Aktivität bilden.

Die Erfindung betrifft auch einen Biochip zur Diagnose von virusinfizierten Zellen, wobei sich eine ausgewählte Anzahl von unterschiedlichen biologischen Wirkstoffen jeweils mit einer biologischen Aktivität auf dem Biochip befinden, das zur Ermittlung eines individuellen Profils der Erkrankung dient, wobei die Wrkstoffe ausgewählt sind aus einer Gruppe von Stoffen bestehend aus Histonen, kovalent modifizierten Histonen, histonähnlichen Polypeptiden und biologisch aktiven Sequenzen der Histone und histonähnlichen Peptide.

## Beschreibung

Die Erfindung betrifft einen biologisch aktiven Wirkstoff insbesondere zur Frühdiagnose und/oder Präventivtherapie von virusinfizierten lebenden Zellen und ein Biochip zur Durchführung der Diagnose.

Viren sind die Erreger für eine Großzahl an unterschiedlichen Krankheiten. Sie lassen sich prinzipiell in zwei große Klassen einteilen; die DNA- und RNA-Viren. Zu den DNA-Viren zählt z.B. die Familie der Herpes-Viren, u.a. das Epstein-Barr-Virus (EBV) und das Pocken-Virus. Zur Klasse der RNA-Viren gehören das Retrovirus HIV (Human Immunodeficiency Virus) oder auch die Masern- und Influenza-Viren. Mitglieder beider Klassen stellen für den Menschen ein großes gesundheitliches Risiko dar. Im Gegensatz zu bakteriellen Erkrankungen, die sich gewöhnlich mit Antibiotika behandeln lassen, gibt es gegen Viren keine spezifischen erfolgreichen Behandlungsmethoden. Meistens erweist sich der frühzeitige Nachweis der Viren als äußerst schwierig und ist indirekt über die Antwort des Immunsystems möglich, die sich in vielen Fällen erst nach Wochen einstellt. Das Immunsystem wird nachfolgend meist sich selbst überlassen, weil keine effektive Virus-Bekämpfungsmethode vorhanden ist, wie im Fall von EBV, das zur infektiösen Mononukleose führen kann, oder es wird das Immunsystem mit bekannten Medikamenten lediglich unterstützt.
Aufgabe der Erfindung ist es, einen biologischen Wirkstoff anzugeben, mit dem selektiv die von Viren befallenen Zellen auch bereits zu einem frühen Zeitpunkt unmittelbar nach dem Virusbefall und noch vor der erkennbaren Antwort des Immunsystems auf den Virusbefall und dem Einsetzen des Virus-Replikationszyklus in der Zelle diagnostizierbar sind und eliminiert werden können. Dabei ist der Wirkstoff in therapeutischen Dosen von beispielsweise 0,5 bis 2 µM weitgehend frei von Nebenwirkungen für den betroffenen Organismus und dessen Immunsystem. Mit anderen Worten soll es sich um einen biologischen Wirkstoff handeln, der insbesondere zur Frühdiagnose und/oder zur Präventivtherapie von virusbefallenen lebenden Zellen und insbesondere bei Säugetieren und Menschen geeignet ist.

Die Aufgabe wird nach den Merkmalen des Anspruches 1 dadurch gelöst, dass der biologisch aktive Wirkstoff wenigstens einen Bestandteil enthält, der ausgewählt ist aus einer Gruppe von Stoffen, bestehend aus Histonen, kovalent modifizierten Histonen, histonähnlichen Polypeptiden und biologisch aktiven Sequenzen der Histone und histonähnlichen Peptide. So kann es erfindungsgemäß vorteilhaft sein, die Wirksamkeit von bekannten Virostatika durch die Zugabe von erfindungsgemäßem Wirkstoff zu unterstützen oder zu erhöhen, so dass mit geringeren Mengen von Virostatika ausgekommen werden kann.

Zur Diagnose und Therapie im Humanbereich besteht der biologische Wirkstoff oder er enthält vorteilhafter Weise rekombinantes humanes Histon H1, oder wenigstens einen Histon H1-Subtyp H 1.0, H 1.1, H 1.2, H 1.3, H 1.4, H 1.5, H 1.t und H 1.x oder ihren biologisch aktiven Abschnitt.

Der erfindungsgemäße Wirkstoff kann aber auch aus einer ausgewählten Anzahl von verschiedenen biologisch aktiven Histonen und/oder histonähnlichen Polypeptiden und/oder deren biologisch aktiven Abschnitten bestehen.

Unter biologischer Aktivität wird hier insbesondere ein Sequenzabschnitt des ausgewählten erfindungsgemäßen Wirkstoffes mit einer biologischen Aktivität gegen Zellmembranen von virusbefallenen Zellen verstanden.

Vorteilhafte Ausführungen der Erfindung offenbaren die Merkmale der Unteransprüche. Dabei betrifft die Erfindung auch einen Biochip zur Auswahl des erfindungsgemäßen biologischen Wirkstoffes mit maximaler biologischer Aktivität gegen virusbefallene Zellen im Organismus eines Menschen oder Säugetieres, der seine jeweiligen individuellen Eigenschaften berücksichtigt und dadurch erst eine optimale Diagnose und/oder Therapie ermöglicht.

Auf dem Biochip zur erfindungsgemäßen Diagnose von virusinfizierten Zellen befinden sich eine ausgewählte Anzahl von unterschiedlichen biologischen Wirkstoffen jeweils mit einer biologischen Aktivität auf dem Biochip, das zur Ermittlung eines individuellen Profils der Erkrankung dient, wobei die Wirkstoffe ausgewählt sind aus einer Gruppe von Stoffen bestehend aus Histonen, kovalent modifizierten Histonen, histonähnlichen Polypeptiden und biologisch aktiven Sequenzen der Histone und histonähnlichen Peptide. Die Immobilisierung der ausgewählten Wirkstoffe auf der Chip-Matrix kann dabei durch PEG-Sequenzen, Oligopeptide, Oligopeptide mit einem N-terminalen Cystein, Gold oder Antikörper erfolgen.

Der Erfindung liegt die Beobachtung zugrunde, dass die Membranen von Zellen schon unmittelbar nach einem Virusbefall und vor dem Einsetzen des Virus-Replikationszyklus in den Zellen charakteristische Änderungen erfahren modifiziert werden, die von dem erfindungsgemäßen Wirkstoff, insbesondere H1 oder einem biologisch aktiven Abschnitt von H1, unabhängig von der Art des Virus erkannt werden.

Hiermit ist erfindungsgemäß erstmals eine Diagnose und/oder Therapie an virusbefallenen Zellen eines Organismus bereits zu einem Zeitpunkt möglich, wo sich noch keine Krankheitssymptome zeigen und noch keine sichere Antwort des Immunsystems erkennbar ist.

Durch den erfindungsgemäßen Wirkstoff ist damit erstmals eine prophylaktische Diagnose an Personen möglich, die sich möglicherweise einer Virusinfektion ausgesetzt haben und bei einem positiven Befund bereits therapiert werden können, bevor die virusbedingte Krankheit zum Ausbruch gekommen ist. Auch ohne eine Frühdiagnose kann auch bei einem bloßen Verdacht auf einen Virusbefall durch den erwägten oder möglich gewesenen Kontakt mit erkrankten Personen oder Tieren eine Präventivtherapie mit dem erfindungsgemäßen Wirkstoff sicherheitshalber möglich sein. Das gilt insbesondere für Personen oder Tiere mit geschwächtem Immunsystem.

Eine derart frühzeitige Therapie hat den wesentlichen Vorteil, dass das Immunsystem der betreffenden Person noch weitgehend intakt ist und bei der Therapie mit dem erfindungsgemäßen Wirkstoff eine unterstützende Funktion entfalten kann.

Die charakteristischen Änderungen oder Modifikationen an der von einem Virus befallen Zellmembran, bereits unmittelbar nach dem Virusbefall, können durch den Kontakt der Zellmembran mit dem Virus und durch sein Eindringen in die Zelle bedingt sein oder haben seinen Grund in Stoffwechseländerungen der Zelle nach einem Virusbefall, die zu Veränderungen an der Zellmembran führen, wobei diese Veränderungen von dem erfindungsgemäßen Wirkstoff erkannt werden. Die Kontaktierung des erfindungsgemäßen Wirkstoffes an einer virusbefallenen Zelle muss nicht einen bestimmten Bindungspartner an der Zellmembran zur Voraussetzung haben. Dies deutet auch darauf hin, dass der erfindungsgemäße Wirkstoff erst durch Bindung des Wirkstoffes an die Zellmembranoberfläche diese in einer Weise schädigt, dass die virusbefallene Zelle lysiert. Die ausreichende Aktivitätsgröße zur Abtötung einer virusbefallenen Zelle kann sich auch danach einstellen, dass durch den ersten Kontakt eines aktiven Wirkstoffbestandteiles an der modifizierten Zellmembranoberfläche eine Kooperativität mit weitere biologisch aktiven Wirkstoffen einsetzt oder begünstigt wird, die miteinander einen biologisch wirksamen Komplex mit gesteigerter biologischer Aktivität bilden.

Die Kooperativität kann erfindungsgemäß begünstigt und/oder beschleunigt werden indem der Wirkstoff nicht nur aus einer Histon-Art und/oder dessen aktiven Abschnitt besteht, sondern eine ausgewählte Anzahl von verschiedenen Histonen und/oder dessen aktiven Abschnitten umfasst.

Mit einem erfindungsgemäßen Biochip lassen sich die biologischen Aktivitäten von Histonen und histonähnlichen Polypeptiden und ihren aktiven Sequenzabschnitten gegenüber individuellen virusinfizierten Zellen leicht ermitteln, um ein individuelles Wirkungsprofil des betreffenden Patienten zu erhalten.

Die Wirksamkeit von beispielsweisen Wirkstoffen, gemäß der Erfindung, wird nachstehend anhand von Versuchen näher beschrieben.

Die folgenden Zeichnungen belegen die durchgeführten Versuche und zeigen, dass Histone virusinfizierte Zellen in vitro abtöten können. Sie zeigen in einzelnen:
- Fig. 1:: Die Überlebensrate von EBV-infizierten humanen B-Lymphozyten nach Inkubation mit rh H 1.3 in Abhängigkeit von der mikromolaren Histonkonzentration.
- Fig. 2:: Absterberate von nichtinfizerten PBMC im Vergleich zu EBNA-positiven Burkitt-Lymphomzellen (Daudi) in Abhängigkeit von der mikromolaren Histon-Konzentration rh H 1.3 im Bereich von 0 bis 10 µM.
- Fig. 3:: Überlebensrate von nichtinfizerten PBMC im Vergleich zu AML-Zellen (U-937) in Abhängigkeit von der mikromodularen Konzentration von H 1.3 im Bereich von 0 bis 7 µM.

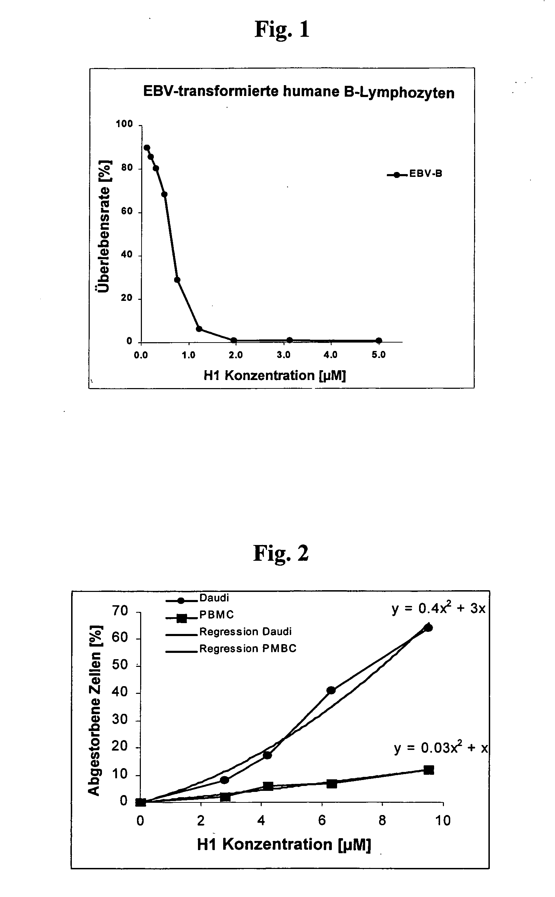

In Fig. 1 wurden EBV(Epstein-Barr-Virus)-infizierte humane B-Lymphozyten verwendet. Durch Inkubation von virusinfizierten Zellen in Gegenwart von rhH 1.3 (rekombinanten humanen H1-Subtyp H 1.3) unterschiedlicher Konzentration kann man eine Lyse und damit verbundenes Absterben der infizierten Zellen beobachten.

Die benötigte Histon-Konzentration ist gering. Schon ab einer H1-Konzentration unter 1 µM können mehr als 50% der Zellen lysiert werden. Konzentrationen ab 2 µM bewirken vollständiges Absterben der Lymphozyten. Der therapeutische Bereich liegt damit hier bei H 1.3-Konzentrationen von 0,5 bis 2,0 µM.

Der Lysevorgang kann mit der Wirkung von Histonen an der durch Virusbefall modifizierten Membran, insbesondere Phospholipidmembran, erklärt werden. So konnten überraschenderweise Histone, die ursprünglich nur im Zellkern gefunden wurden, in der Plasmamembran vieler Zellen, unter anderem auch virusinfizierter Zellen nachgewiesen werden. Es konnte weiter gezeigt werden, dass Histon H1 an virusspezifische Proteine mit hoher Affinität bindet und für den Replikationszyklus des Virus wichtig ist.

Der Einbau von Fremdproteinen wie Histonen in die Membran schädigt die Membranintegrität, so dass lösliche Proteine, Ionen und andere Zellbestandteile unkontrolliert nach außen entweichen können und die Zelle dadurch abstirbt.

Der Einbau von Histonen kann antigenvermittelt sein, so dass ein Zelloberflächenpräsentiertes Antigen als Bindungsprotein für das Histon dienen kann.

Infiziert das Virus eine Zelle, produziert die Zelle im Laufe ihres virusgesteuerten Zyklus virus- und zellspezifische Antigene, die an die Zelloberfläche gelangen und dort präsentiert werden. Im Falle des EBV werden sogenannte EBNA-Antigene synthetisiert. In vitro konnte gezeigt werden, dass Histon H1 mit hoher Affinität an diese Proteine bindet.

Denkbar wäre auch eine rezeptorfreie Histonbindung an die Membran, wobei diese allein durch das Eindringen des Virus in die Zelle derart verändert wird, dass sie eine Bindung des Histons und nachfolgende Lyse zulässt.

Die charakteristische Abhängigkeit der Zellsterblichkeit von der logarithmisch aufgetragenen Histon-Konzentration deutet hierbei auf einen kooperativen Effekt hin. Das bedeutet, dass der Lysevorgang erst ab einer bestimmten Konzentration an gebundenen Histonen einsetzt, was die weitere Histonbindung erleichtert und die Lyse dadurch beschleunigt. Der gleiche Effekt ist auch für homogene Histonlösungen anderer Typen, wie z.B. H2, H3 und H4 und für heterogene Gemische der Histone zu erwarten.

Die Lyse von virusinfizierten Zellen ist hochspezifisch. Nichtinfizierte Zellen werden von Histonen nicht oder nur in sehr geringem Maße lysiert.

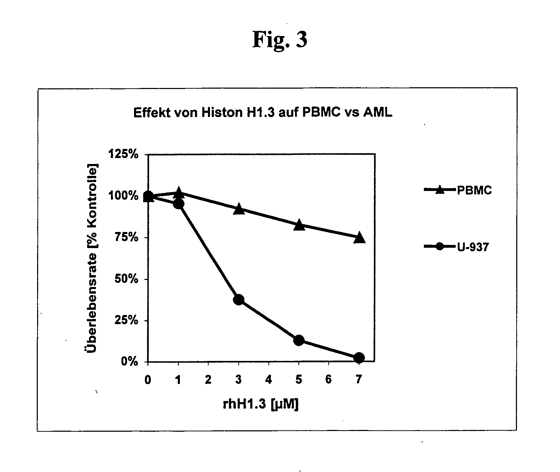

Die nachstehenden Fig. 2 und 3 zeigen als Negativkontrollen die geringe Wirkung von Histon rh H 1.3 gegenüber nichtinfizierten Lymphozyten, in dem Fall peripheren Blutmonozyten (PBMC).

Man erkennt an der flach ansteigenden Kurve y = 0,03 x² + x, dass nichtinfizierte periphere Blutlymphozyten (PBMC) deutlich weniger angegriffen werden als die durch EBV-Infektion generierte Burkitt-Lymphom-Zellinie Daudi, deren Absterberate durch die vergleichsweise steil ansteigende Kurve y = 0,4 x² + 3x gekennzeichnet ist.
Die Kurven der Fig. 2 zeigen einen parabolischen Anstieg der Zellsterblichkeit der virusinfizierten Zellen in Abhängigkeit von der H 1.3-Konzentration. Die Sterblichkeit von nichtinfizierten PBMC liegt bei hohen H 1.3-Konzentrationen, im Vergleich zu verwendeten Bedingungen bei EBV-infizierten B-Zellen (Fig. 1), von etwa 10 µM nur bei 10%. Unter diesen Bedingungen findet man bei Daudi-Zellen schon eine um die 70%-ige Lyse.

Die Überlebensrate vom PBMC in Fig. 1 beträgt noch bei einer rhH 1.3-Konzentration von 7 µM 75%. Im Unterschied dazu sterben Zellen der akuten myeloischen Leukämie (AML) bei dieser Histon-Konzentration vollständig ab. Leukämie kann durch onkogene Viren wie z.B. HTLV I ausgelöst werden. Hierbei zeigt sich, in Analogie zu EBNA-positiven Burkitt-Lymphomzellen (Fig. 2), dass die lytische Wirksamkeit der Histone unabhängig vom Stadium der Zellen ist. Die Zellen werden erkannt und lysiert, auch wenn sie sich durch eine vorangegangene Virusinfektion bereits zu Lymphom- bzw. Leukämiezellen verändert haben.

Im Gegensatz dazu liegt die Überlebensrate von Phytohämagglutinin(PHA)-aktivierten normalen T-Lymphozyten in der gleichen Größenordnung wie bei den peripheren Blutlymphozyten.

Hämaglutinin gehört zu Glykoproteinen der viralen Lipidmembran, welche für die Adhesion von Viren an Zelloberflächen verantwortlich sind, und gilt deshalb als am meisten virulenter Faktor der Viren. In der Zellbiologie wird PHA häufig für die Stimulierung von peripheren Blutzellen verwendet.

Hier zeigt sich aber, dass die Aktivität der Histone sich nicht selektiv gegen PHAaktivierte Zellen richtet.

Zusammenfassend lässt sich sagen, dass bisher erhaltene Befunden darauf hin deuten, dass die Selektivität der Histonbindung durch die besondere Modifikation von Zellmembranen bedingt ist, wie sie erst in spezifischer Weise durch die Virusinfektion Zellen hervorgerufen wird. Überraschen ist, dass die Histonbindung von der Art des Virus und auch der Art der infizierten Zellen unabhängig zu sein scheint und der Effekt sowohl bei Vertretern der DNA- (z.B. EBV) als auch der RNA-Viren (z.B. HIV) auftritt.

Zu den getesteten Zellen zählen Zellen des Immunsystems wie T- und B-Lymphozyten. Letztere konnten direkt nach einer EBV-Infektion mit Histon H1 abgetötet werden (Fig. 1). Eine längere Infektion mit EBV kann zum bösartigen Burkitt-Tumor führen. Durch Zugabe von Histonen zu Zellen in solchem Stadium konnte ebenfalls eine Zelllyse ausgelöst werden (Fig. 2).

Damit zeigt sich, dass virusinfizierte Zellen, unabhängig vom zurückliegenden Zeitpunkt der Infektion, von Histonen erkannt und beseitigt werden. Für eine EBV-Infektion bietet die Behandlung mit Histonen sowohl Chancen für eine schnelle Diagnose (z.B. von Blutbestandteilen) und Therapie, noch bevor die Entwicklung zum Burkitt-Lymphom oder zur infektiösen Mononukleose (Pfeiffer-Drüsenfieber) führen kann. Bisher gibt es für die EBV-Infektion keine frühen Diagnosemöglichkeiten und keine effektive Therapie.

Ebenfalls bietet die Anwendung von Histonen neue Möglichkeiten in der Diagnose und Therapie von virusinduzierter Leukämie.

Alle Proteine besitzen aktive Zentren, bzw. Bereiche, die für ihre spezifischen Aufgaben notwendig sind. Deshalb können zur Diagnose und Bekämpfung von virusinfizierten Zellen neben nativen oder vollständigen rekombinanten Histonen auch modifizierte Histone, histonähnliche Polypeptide oder Abschnitte daraus eingesetzt werden, bei denen aber die biologisch aktiven Zentren erhalten bleiben. Bei den kovalenten Modifikationen kann es sich um Phosphorylierung, Acetylierung, Methylierung, Ribosylierung oder Ubiquitinylierung handeln.

Eine Möglichkeit zur Diagnose von virusinfizierten Zellen stellen Biochips dar. Ein Biochip ist eine Ansammlung von miniaturisierten Teststellen (Microarrays), die an einem festen Substrat angeordnet sind. Er erlaubt viele gleichzeitige Untersuchungen und ermöglicht dadurch einen großen Durchsatz. So kann ein Biochip Tausende von biologischen Reaktionen in nur einigen Sekunden durchführen. Mit einem speziell gestalteten optischen Detektionssystem können die positiven Reaktionen, d.h. die Orte der Bindung lokalisiert werden.
In unserem Fall können unterschiedliche Histontypen und -subtypen, kovalent modifizierte Histone, histonähnliche Polypeptide oder biologisch aktive Abschnitte daraus, an einen Biochip gebunden werden, die als Bindungsanker für Virusinfizierte Zelle dienen. Dadurch kann man unterschiedliche Zellen vieler Patienten gleichzeitig auf die Affinität zu verschiedenen Komponenten des Wirkstoffs untersuchen, um festzustellen, ob eine Virusinfektion vorliegt. Die Art der Bindung der biologisch aktiven Wirkstoffe an die Chip-Oberfläche kann über PEG-Sequenzen, Oligopeptide, Oligopeptide mit terminalem Cystein, Goldpartikel oder über spezifische Antikörper erfolgen.

## Patentansprüche

1. Biologisch aktiver Wirkstoff, insbesondere zur Frühdiagnose und/oder Präventivtherapie von virusinfizierten lebenden Zellen, wobei seine Wirksamkeit selektiv gegen die Zellmembranen der virus-infizierten Zellen gerichtet ist, die nach dem Virusbefall in charakteristischer Weise entartet modifiziert sind, wobei der Wirkstoff wenigstens einen Bestandteil enthält oder aus ihm besteht, der ausgewählt ist aus einer Gruppe von Stoffen bestehend aus Histonen, kovalent modifizierten Histonen, histonähnlichen Polypeptiden und biologisch aktiven Sequenzen der Histone und histonähnlichen Peptide.

2. Biologischer Wirkstoff nach Anspruch 1, **dadurch gekennzeichnet, dass** der Wirkstoff Histon H1 oder sein biologisch aktiver Abschnitt ist.

3. Biologischer Wirkstoff nach Anspruch 2, **dadurch gekennzeichnet, dass** der Wirkstoff rekombinantes humanes Histon H1 oder wenigstens ein rekombinantes humanes Histon H1-Subtyp H 1.0; H 1.1, H 1.2, H 1.3, H 1.4, H 1.5, H 1.t, H1.x oder dessen biologisch aktiver Abschnitt ist.

4. Biologischer Wirkstoff nach Anspruch 1, **dadurch gekennzeichnet, dass** die ausreichende Größe der biologischen Aktivität zur Abtötung von virusinfizierten Zellen an ihren modifizierten Zellmembranen durch die Kooperativität mit gleichen oder unterschiedlichen aktiven Wirkstoffbestandteilen erzielbar ist, die miteinander einen biologisch wirksamen Komplex mit gesteigerter biologischer Aktivität bilden.

5. Biologischer Wirkstoff nach Anspruch 1, **dadurch gekennzeichnet, dass** die Modifikation der Zellmembran charakterisiert ist durch ein Virus in der Zelle, wobei die biologische Aktivität des Wirkstoffes unabhängig von der Charakteristik Modifikation oder Entartung der Zellmembran ist, die für die Art des Virus charakteristisch ist.

6. Biologischer Wirkstoff nach Anspruch 1, **dadurch gekennzeichnet, dass** die Virus-infizierten Zellen Zellen des Immunsystems sind.

7. Biologischer Wirkstoff nach Anspruch 6, **dadurch gekennzeichnet, dass** die Virus-infizierten Zellen T-Lymphozyten und/oder B-Lymphozyten sind.

8. Biologischer Wirkstoff nach Anspruch 1, **dadurch gekennzeichnet, dass** das Virus ein RNA-Virus ist.

9. Biologischer Wirkstoff nach Anspruch 1, **dadurch gekennzeichnet, dass** das Virus ein DNA-Virus ist.

10. Biologischer Wirkstoff nach Anspruch 8, **dadurch gekennzeichnet, dass** das Virus ein SI-Virus oder ein HI-Virus oder ein Retrovirus ist und die befallenen Zellen Immunzellen sind.

11. Biologischer Wirkstoff nach Anspruch 10, **dadurch gekennzeichnet, dass** die Immunzellen T-Zellen sind.

12. Biologischer Wirkstoff nach Anspruch 9, **dadurch gekennzeichnet, dass** das Virus ein EB-Virus ist und die EB-Virus-befallenen Zellen B-Lymphozyten sind.

13. Biologischer Wirkstoff nach einem der vorstehenden Ansprüche zur Diagnose und/oder Therapie von infektiöser Mononukleose.

14. Biochip zur Diagnose von Virus-infizierten Zellen, **dadurch gekennzeichnet, dass** sich eine ausgewählte Anzahl von unterschiedlichen biologischen Wirkstoffen jeweils mit einer biologischen Aktivität auf dem Biochip befinden, das zur Ermittlung eines individuellen Profils der Erkrankung dient, wobei die Wirkstoffe ausgewählt sind aus einer Gruppe von Stoffen bestehend aus Histonen, kovalent modifizierten Histonen, histonähnlichen Polypeptiden und biologisch aktiven Sequenzen der Histone und histonähnlichen Peptide.

15. Biochip nach Anspruch 14, **dadurch gekennzeichnet dass** die Immobilisierung der Wirkstoffe auf der Chip-Matrix durch PEG-Sequenzen, Oligopeptide, Oligopeptide mit einem N-terminalen Cystein, Gold oder Antikörper erfolgt.
